# EUROPEAN PATENT APPLICATION

(11) **EP 1 106 209 A1**
(43) Date of publication of application: **13.06.2001**
(21) Application number: 00118729.3
(22) Date of filing: 30.08.2000
(51) Int. Cl.: A61N 5/067

(54) **Laser-therapy device**

(30) Priority: 10.12.1999 IT GE990141
(71) Applicant: R.G.M. S.p.A., 16157 Genova (IT)
(72) Inventor: Baratto, Luigi, 16167 Genova (IT); Brescia, Giuseppe, 16039 Sestri Levante (IT); Morasso, Pietro, 16148 Genova (IT); Gregori, Massimiliano, 16157 Genova (IT); Capra, Roberto, 17100 Savona (IT)
(74) Representative: Porsia, Attilio, Dr.

(57) **Abstract**

Device for laser-therapy, including a supplying unit (2), a laser generator (1), a processing unit (3) and an application peripheral unit (4); the laser generator (1) emits by pulses under the control of a microprocessor control unit (2) which allows to change both the "duty cycle" meant as ratio between the emission total time and the total functioning time of the emission itself (1-99%) and the pulses repetition frequency (50:5000 Hz).

## Description

The present invention refers to the devices for laser-therapy.

Said devices include devices able to emit a coherent light radiation with wavelengths included between 400÷1500 nm; their use is well-known in the treatment of pathologies both acute and chronic. In particular, laser-therapy treatment are held when it is necessary to increase the recovery speed in the reparation of damaged tissues, to offer relief in case of painful syndromes, or to reduce the inflammations.

Normally, the action of the laser-therapy device is finalized to the transfer of energy to the tissues, through the supplying of energy both continuos and pulsive. This kind of approach has undoubted advantages, but is not free from disadvantages. Generally the most frequent problem is concerned with the warming of the areas near the ones subjected to the treatment which can cause damages such that they drastically reduce the global efficacy of the therapy; moreover the power used today are such that the eventual accidental damages to particularly sensitive tissues, like for instance the retina, can be of great extent.

Aim of the present invention is then to offer a device for laser-therapy where the primary aim is the transfer of a signal fit to stimulate the physiological reaction of the tissue specifically interested instead of transferring locally to them a considerable thermal energy.

Object of the present invention is then a device for laser-therapy, including a supplying unit, a laser generator, a processing unit and an application peripheral unit characterized in that the laser generator has a very limited power, emits by pulses and both its repetition frequency and the "duty cycle" of its emission can be suitable controlled to offer a specific tissue stimulation.

In an embodiment form with immediate application the emission wavelength has been chosen around 635÷680 nm, the power of the emission has been valued 3÷5 mW, the pulses repetition frequency can be selected between 50 and 5000 Hz and the "duty-cycle" of the emission is adjustable between 1 and 99%.

Further advantages and features will become evident by the following description of an embodiment form of the laser-therapy device according to the present invention, to be considered as an illustrative and not limitative description, with reference to the enclosed drawings, where:
Figure 1 is a diagram of the device according to the invention; and
Figure 2 is a graph showing the functioning cycle of the invention device.

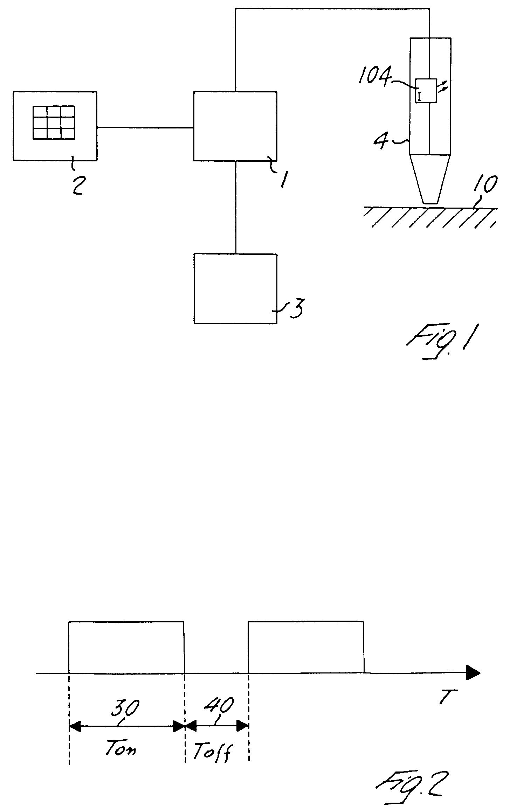

In figure 1 is schematically shown the laser-therapy device according to the invention; with the numeral 4 is shown an applicator handpiece where the laser generator 104 is housed, a laser diode emitting radiation in the range included between 635 and 680 nm, with an emitting power included between 3 and 5 mw; the generator 104 is connected to a processing and control unit 1 able to pilot the radiation emission; the supplying unit 3 supplies the system which is controlled by the operator through the control keyboard/display 2.

In figure 2 is exemplified an emission pulses sequence of the device according to the invention. As it is noticeable from the graph, the emission time 30 and the interruption time 40 are each other unsymmetrical. Normally it can be selected an emission time included between 0.1 and 9.9 ms, and interruption times between 0.1 and 9.9 ms. Both the times can be selected by the operator by steps of 0.1 ms. From that result the variability ranges both of the pulses repetition frequency (50÷5000 Hz) and the "duty-cycle" (1÷99%) .

In the instrument according to the invention it has been chosen to transfer to the tissue a stimulation signal instead of a considerable quantity of energy, as in the traditional systems. This kind of choice allows to considerably reduce the power of the laser source; the use of a limited power source makes from one side the device more advantageous from the energy point of view, and from the other side increases the safety of its use, considering the risks coming from the unintentional sighting to particularly sensitive tissues.

Here are reported two protocols which has been successfully tested on the patients and refer to the most common therapeutic applications.

| Emission time (ms) | Interruption time (ms) | Frequency (Hz) | Duty cycle (%) | Energy (J/min) |
|---|---|---|---|---|
| 0,4 | 0,6 | 1000 | 40 | 0,011 |
| 1,8 | 0,2 | 500 | 90 | 0,016 |

The topical eutrophic applications, fit to help the absorption of edema or the healing of wounds, are locally made, while those for the therapy of the myofascial pain can be made in the zones corresponding to those where the algesic symptomatologies are, for instance according the principles of the Chinese medicine.

The applications have preferably a duration of less than 5 minutes, and generally included between 1 and 3 minutes.

## Claims

1. A laser-therapy device, including a supplying unit (3), a laser generator (104), a processing unit (1) and an application peripheral unit (4) characterized in that the laser generator (104) emits at low power level, by pulses with changeable frequency and duration, and its duty-cycle is controlled though said processing unit (1).

2. A laser-therapy device according to claim 1, characterized in that it transfers to the patient a signal fit to stimulate the cell and tissue reaction.

3. A device according to claims 1 and 2, where the emission time of said generator (1) is within 0,1 and 9,9 ms.

4. A device according to claims 1, 2 or 3, where the interruption time of said generator (1) is within 0,1 and 9,9 ms.

5. A device according to claims 1, 2, 3 or 4 where the emission time and the interruption time of said generator are generally different from each other.

6. A device according to any preceding claim 1 to 5, where the pulses emission frequency of said generator (1) is within 50 and 5000 Hz.

7. A device according to any one of the preceding claims, where the emission power of said generator is in the range of 1-10 mW, and preferably within 3 and 5 mW.
